# EUROPEAN PATENT APPLICATION

(11) **EP 4 628 158 A1**
(43) Date of publication of application: **08.10.2025**
(21) Application number: 23898390.2
(22) Date of filing: 01.12.2023
(51) Int. Cl.: A61N 2/02, A61N 2/00

(54) **PULSED ELECTROMAGNETIC FIELD GENERATION APPARATUS AND DRIVING METHOD FOR SAME APPARATUS**

(30) Priority: 01.12.2022 KR 20220165764
(71) Applicant: EntWick Co., Ltd., Yongin-si, Gyeonggi-do 16914 (KR)
(72) Inventor: JEONG, Jae Joon, Seoul 03323 (KR)
(74) Representative: Cabinet Chaillot
(86) International application number: PCT/KR2023/019700
(87) International publication number: WO 2024/117867

(57) **Abstract**

The present invention relates to a magnetic field generation unit, which is a key component for treatment in various treatment devices using a pulsed electromagnetic field, and to a driving method therefor, wherein the magnetic field generation unit includes a magnetic field generation module equipped with two or more coil electromagnets and inner and outer plates of ferromagnetic material to maximize the attraction and repulsion between the electromagnets, and this magnetic generation module is rearranged along a central axis in a horizontal or vertical space, so as to transmit a magnetic force over a long distance at low power through the attraction and repulsion between the magnetic field generation module and a magnetic field generation module on the other side.

## Description

### [Technical Field]

The present invention relates to a pulsed electromagnetic field generation apparatus and a method of driving the apparatus, and more particularly, to an electromagnet device of a pulsed electromagnetic field treatment device in which magnetic field generation modules to which inner and outer plates of a ferromagnetic material are applied are provided as a magnetic field generation unit which is a core component for treatment in various treatment devices that utilize, for example, a pulsed electromagnetic field, to maximize attractive and repulsive forces between two or more coil electromagnets and electromagnets, and a magnetic force is transmitted to a long distance at low power through attractive and repulsive forces between the magnetic field generation modules on opposite sides by rearranging the magnetic field generation modules so that the magnetic field generation modules are aligned with a central axis in a horizontal or vertical space, and a method of driving the electromagnet device.

### [Background Art]

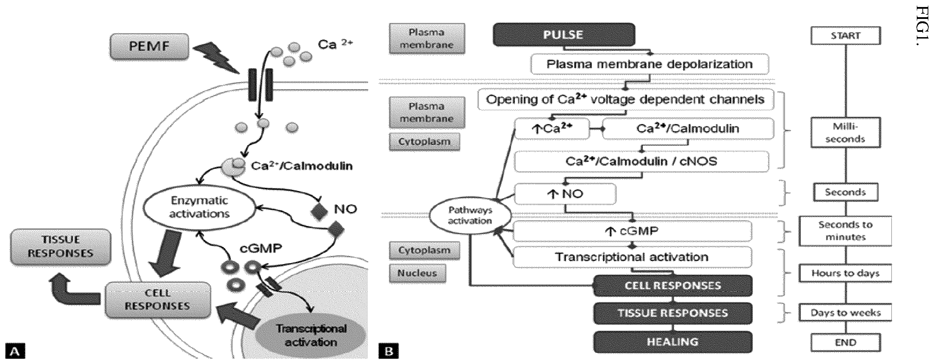

Pulsed electromagnetic field therapy, which is one non-invasive treatment, is being applied to many treatment devices as its treatment mechanism has been revealed through many studies. When a pulsed electromagnetic field is generated in the form of a constant low-frequency pulse as described in a mechanism in FIG. 1 and applied to a cell, a depolarization phenomenon occurs in a specific part of a cell membrane, which causes Ca²⁺ ions in the blood to flow into the cell and increase the concentration of nitric oxide (NO) in the cytoplasm, cyclic guanosine monophosphate (cGMP) is generated in the nucleus due to the increased concentration of nitric oxide (NO), and thus cell and tissue are activated.

That is, when microcurrents are generated in cells through a pulsed electromagnetic field, an absorption rate of oxygen and minerals in cells increases, toxins within cells decrease, and metabolism of cells is activated. This has been confirmed through numerous research papers, and this is being used to treat numerous wounds and pain due to its clinical effects of promoting bone formation and reducing swelling and pain.

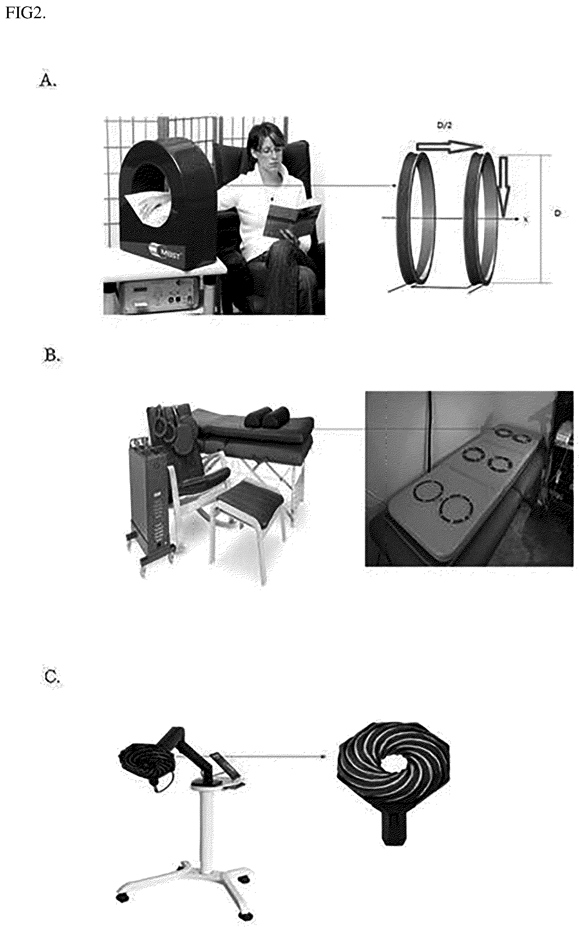

However, most current treatment devices using a pulsed electromagnetic field use loop-type coils to generate an electromagnetic field. However, since it is structurally difficult to transmit a magnetic force to a long distance, high power should be applied to the coils. As a result, the size of the system increases, making it difficult to carry, and thus there is a limitation in application as a personal treatment device. For example, MBST's product described in FIG. 2 applies the principle of the Helmholtz Coil, and thus, when a diameter of the loop coil is D, a loop coil of the same size should be applied at a D/2 position, which increases the size of the treatment device and requires high power to transmit a sufficient magnetic force to the center of the coil, which requires a relatively large control facility for current control. Medtech's products also use a loop-shaped coil shape, so the size of the electromagnetic field generation unit is large and high power is required, and it is difficult to expect three-dimensional magnetic force transmission due to the flat two-dimensional arrangement. Further, SE Therapies' products use a single electromagnetic field generation module, so there is no interaction between coils, and because a plurality of coils are combined in a spiral shape to transmit a magnetic force to a long distance, the problems arise that the size increases and high power is required.

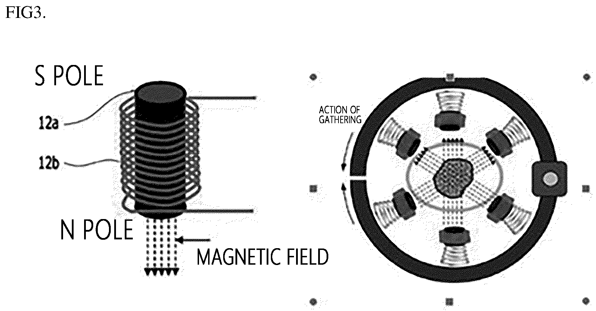

Further, as illustrated in FIG. 3, in the conventional related art patent technology, a technology of radially arranging a plurality of single coil electromagnet modules to induce an attractive force with an electromagnet module on the other side is proposed, but there is a problem in that a relatively large amount of power is required to induce an attractive force with an electromagnet on the other side that is spaced a long distance away.

In summary, the devices of the conventional technologies are relatively large in size because a loop-shaped coil is applied to the devices, and require high power for long-distance magnetic force transmission, and for this, the capacity and size of the control device become large, making it difficult to develop applications for portable personal treatment devices. Further, in the case of modules to which a single coil electromagnet is applied, relatively high power is required to induce an attractive force with the other electromagnet module positioned at a long distance, and the control device for controlling the power also becomes large.

Meanwhile, the pulsed electromagnetic field (PEMF) treatment is a treatment method that aids cell regeneration and relieves inflammation by applying a pulsed electromagnetic field to a target and has been widely used since the US Food and Drug Administration approved it since 1979 for fracture healing related to malunion. Recently, as various clinical trials and studies are being conducted, the PEMF treatment is being applied to the treatment of various diseases (e.g., for the treatment of muscle pain, osteoporosis, arthritis, etc.). Further, the magnetic force usually with an intensity of several to several hundred mT and having a frequency of several hundred Hz or less is applied to the target in the form of a constant pulse.

The pulsed electromagnetic field generation unit applied to the conventional pulsed electromagnetic field treatment device is formed such that the coil is in the form of a loop wire, and the magnetic force decreases rapidly with distance, and a large capacity of power is required to transmit the magnetic force to a long distance. Several copper coils are wound, a current is applied thereto, and an electromagnetic force F generated therefrom is utilized. Due to this problem, high power is required to transmit the magnetic force to a long distance, and as a result, the size of the control unit and the size of the product are also increased.

Further, the pulsed electromagnetic field generation unit applied to the conventional pulsed electromagnetic field treatment device is formed with a coil in the form of a loop wire, and thus the magnetic field is generated by passing through the center of the coil. There is no problem in the case of tissues positioned far away to be treated, but when the purpose of treatment is tissues positioned close by, such as skin, it is desirable to limit the area where the magnetic force is generated. For example, when the treatment method is applied for regeneration of facial skin tissue, an unintentional magnetic field may affect the brain. However, the conventional technology has a problem in that the range of magnetic force generation cannot be limited because of the form of a loop wire coil.

The pulsed electromagnetic field changes the magnetic force by changing the voltage applied to the coil. That is, while the resistance of the coil is fixed, the amount of current is controlled by adjusting the strength and frequency of the voltage, and as a result, the strength and frequency of the magnetic field B are also changed, which can induce various waveforms. According to the strength and waveform of the magnetic field, there are various therapeutic effects, and clinical trials are being conducted on the therapeutic effects.

Referring to the paper "Veterinary applications of pulsed electromagnetic field therapy" James S. Gaynora, Sean Hagbergb, Blake T. Gurfeinc, 2018 presented as the related art document, a waveform is a digital A waveform proposed in a bone growth stimulator, and has an asymmetrical characteristic in which the waveform is repeated at 15 Hz intervals with 5 ms bursts at 200 µs and 20 µs intervals, and transmits an electric field E of 5 V/m. In addition, a B waveform in analog form is used in the proposed device to reduce swelling and pain, and has the characteristic of short waves with 2ms bursts at 27.12 MHz intervals repeated at 2Hz intervals, and transmits an electric field E of 10 V/m. In this way, a waveform of a voltage applied to the coil affects the strength of the electric field E and magnetic field B transmitted to a long distance. As described above, the polarity direction of the magnetic force should alternate at regular intervals without energy loss to transmit the magnetic force to a long distance with low power and maximize therapeutic effects. However, in the conventional method presented in the paper, heat is generated in the coil and energy is consumed due to the counter electromotive force of the coil by continuously applying voltage changes. That is, there is a problem of low energy efficiency.

A treatment device using a pulsed electromagnetic field may have a plurality of magnetic field generation units. A configuration of a circuit that generates a pulsed electromagnetic field may be composed of a control unit that controls the circuit, a magnetic polarity control unit that controls a direction of a current, such as an H-bridge or a digital-analog converter (DAC), a power supply unit that amplifies the power consumption and provides the power to a polarity control unit, such as a DC/DC, a battery management system (BMS), or operational amplifier (OP AMP), and an electromagnetic field generation unit composed of a coil for generating a magnetic force. As described in the waveform of the voltage applied to the magnetic field generation unit, since the generated magnetic field has a constant frequency, when two or more magnetic field generation units are used and the effect of mutual superposition and cancellation between the generated magnetic forces is expected, it is advantageous to match the synchronization of the magnetic field generation units.

In the conventional technology, the control unit such as a central processing unit (CPU) and a Micom controls a plurality of magnetic field generation units, and thus the synchronization between the magnetic field generation units does not match, which causes asynchronous sections, and as a result, the efficiency of superposition and cancellation between the magnetic field generation units is reduced. As a practical example, when four magnetic field generation units are controlled by one Micom, the magnetic generation time difference (asynchronous section) between adjacent magnetic force generation units may be several milliseconds or more.

### [Related Art Documents]

### [Patent Documents]

Korean Patent Registration No. 10-1695096 (January 4, 2017)
Korean Patent Registration No. 10-1768795 (August 9, 2017)
Korean Laid-open Patent Publication No. 10-2021-0106120 (August 30, 2021)

### [Non-patent Document]

Paper: "Veterinary applications of pulsed electromagnetic field therapy" James S. Gaynora, Sean Hagbergb, Blake T. Gurfeinc, 2018

### [Detailed Description of Invention]

### [Technical Problem]

Embodiments of the present invention are directed to providing an electromagnet device of a pulsed electromagnetic field treatment device, in which magnetic field generation modules to which inner and outer plates of a ferromagnetic material are applied are provided as a magnetic field generation unit which is a core component for treatment in various treatment devices that utilize, for example, a pulsed electromagnetic field, to maximize attractive and repulsive forces between two or more coil electromagnets and electromagnets, and a magnetic force is transmitted to a long distance at low power through attractive and repulsive forces between the magnetic field generation modules on opposite sides by rearranging the magnetic field generation modules so that the magnetic field generation modules are aligned with a central axis in a horizontal or vertical space, and a method of driving the electromagnet device.

Embodiments of the present invention are also directed to providing a pulsed electromagnetic field generation apparatus that is a pulsed electromagnetic field generation module (or device, component, etc.) applicable to, for example, a pulsed electromagnetic field (PEMF) treatment device and can control the long-distance magnetic force transmission and magnetic field generation area using a pair-type electromagnet, which includes a pair-type electromagnet unit, that is, a pair-type electromagnet device, using a multilayer printed circuit board (PCB) pattern, an electromagnet unit that utilizes a permanent magnet and a coil, and a pulsed electromagnetic field generation unit through permanent magnet rotation, and which is capable of performing operations such as voltage control frequency and pulsed electromagnetic field synchronization using a trigger switch, and a method of driving the apparatus.

### [Technical Solution]

A pulsed electromagnetic field generation module (or unit, device, element, component, etc.) according to one embodiment of the present invention may be an electromagnet unit composed of two or more coil electromagnets, and the electromagnet unit may transmit a magnetic force to a predetermined long distance exceeding a reference value by applying repulsive and attractive forces of the coil electromagnets.

The coil electromagnet may include a winding coil wound around a cylindrical frame, a pair-shaped ferromagnetic core that is positioned at the center of the winding coil and transmits a magnetic field generated by a current applied to the winding coil in a predetermined direction, one ferromagnetic plate that is provided at one side of the ferromagnetic core and transmits the magnetic force transmitted from the ferromagnetic core to a predetermined long distance, and the other ferromagnetic plate that is provided at the other side of the ferromagnetic core and connects a plurality of types of ferromagnetic cores to each other to increase the magnetic force of the one plate. The coil electromagnet may further include a plurality of permanent magnets with a direction of a magnetic force determined between a plurality of coil electromagnets to transmit the magnetic force to a longer distance.

The one plate may include a plurality of plates each connected to one sides of the plurality of types of ferromagnetic cores, and the plurality of plates may be formed to be separated from each other with a predetermined interval.

The other plate may connect the other sides of the plurality of ferromagnetic cores and may be formed as a single plate facing the one plate.

The pulsed electromagnetic field generation unit may modulate a frequency of a pulse signal having a predetermined frequency and amplitude to transmit the magnetic force to a predetermined long distance.

The pulsed electromagnetic field generation unit may include at least two electromagnet units and generate a repulsive force from each of the electromagnet units to transmit the magnetic force to the predetermined long distance.

The permanent magnet may be positioned between the coil electromagnets to transmit the magnetic force to a longer distance by utilizing the repulsive force with an adjacent coil electromagnet.

Further, a method of driving an electromagnetic field generation module according to one embodiment of the present invention includes an operation of setting the polarity of a plurality of coil electromagnets to match a starting point at which attractive and repulsive forces are generated with respect to an electromagnetic field generation module on the other side or an adjacent side, and an operation of controlling start and stop time points of all the coil electromagnets with a trigger switch.

Furthermore, a pulsed electromagnetic field generation apparatus according to one embodiment of the present invention includes an electromagnet unit configured to generate attractive and repulsive forces between a plurality of coil electromagnets, each of which is formed by winding a coil around a core, and adjust a magnetic force (F) generated by the plurality of coil electromagnets, and an electromagnet operation switch unit configured to adjust at least one of a voltage and frequency applied to each of the plurality of coil electromagnets.

The electromagnet unit may include a first electromagnet unit which includes a pair of a first coil electromagnet and a second coil electromagnet and generates attractive and repulsive forces between the first coil electromagnet and the second coil electromagnet, and a second electromagnet unit which includes a pair of a third coil electromagnet and a fourth coil electromagnet, generates attractive and repulsive forces between the third coil electromagnet and the fourth coil electromagnet, and generates attractive and repulsive forces between the first electromagnet unit and the second electromagnet unit.

The pulsed electromagnetic field generation apparatus may further include an electrode control unit configured to change a magnetic polarity by changing a direction of a current applied to each of the first electromagnet unit and the second electromagnet unit, and control the magnetic polarity to be changed in an AC form.

The electromagnet operation switch unit may include a trigger switch that simultaneously turns on or off the first electromagnet units and the second electromagnet unit.

The electromagnet unit may include one plate formed of a plurality of plates separated at one side of the first coil electromagnet and the second coil electromagnet, and the other plate formed of a single plate formed by connecting one side surfaces of the first coil electromagnet and the second coil electromagnet to each other at the other side of the first coil electromagnet and the second coil electromagnet.

The electromagnet unit may include a permanent magnet that is provided between the one plate of the first coil electromagnet and the one plate of the second coil electromagnet to transmit the magnetic force to a long distance.

The plurality of coil electromagnets may be formed using a conductive pattern formed on a multilayer printed circuit board (PCB).

Further, a method of driving a pulsed electromagnetic field generation apparatus according to one embodiment of the present invention includes generating, by an electromagnet unit, attractive and repulsive forces between a plurality of coil electromagnets, each of which is formed by winding a coil around a core, and adjusting a magnetic force (F) generated by the plurality of coil electromagnets, and adjusting, by an electromagnet operation switch unit, at least one of a voltage and frequency applied to each of the plurality of coil electromagnets.

The adjusting of the magnetic force may include generating, by a first electromagnet unit including a pair of a first coil electromagnet and a second coil electromagnet, attractive and repulsive forces between the first coil electromagnet and the second coil electromagnet, and generating, by a second electromagnet unit including a pair of a third coil electromagnet and a fourth coil electromagnet, attractive and repulsive forces between the third coil electromagnet and the fourth coil electromagnet, and generating attractive and repulsive forces between the first electromagnet unit and the second electromagnet unit.

The method may further include changing, by an electrode control unit, a magnetic polarity by changing a direction of a current applied to each of the first electromagnet unit and the second electromagnet unit, and controlling the magnetic polarity to be changed in an AC form.

The adjusting of the at least one of the voltage and frequency may include simultaneously turning on or off, by a trigger switch constituting the electromagnet operation switch unit, the first electromagnet unit and the second electromagnet unit.

The electromagnet unit may include one plate formed of a plurality of plates separated at one side of the first coil electromagnet and the second coil electromagnet, and the other plate formed of a single plate formed by connecting one side surfaces of the first coil electromagnet and the second coil electromagnet to each other at the other side of the first coil electromagnet and the second coil electromagnet.

The electromagnet unit may include a permanent magnet that is provided between the one plate of the first coil electromagnet and the one plate of the second coil electromagnet to transmit the magnetic force to a long distance.

The plurality of coil electromagnets may be formed using a conductive pattern formed on a multilayer PCB.

### [Advantageous Effects]

According to embodiments of the present invention, the size and power consumption of an electromagnetic field generation module for generating a pulsed electromagnetic field can be minimized, thereby minimizing the size of a treatment device, and the start and stop time points of each coil electromagnet can be synchronized to maximize the interaction time of a plurality of coil electromagnetic field generation modules, thereby ultimately increasing therapeutic effects.

### [Description of Drawings]

FIG. 1 is a diagram for describing a cell activation mechanism using a pulsed electromagnetic field.
FIG. 2 is a diagram for describing treatment devices using pulsed electromagnetic fields made by MBST Co. (see FIG. 2A), Medtech Co. (see FIG. 2B), and SE Therapies Co. (see FIG. 2C), and shapes of loop coils inside the devices.
FIG. 3 is a diagram for describing the shape of a conventionally proposed coil electromagnet.
FIG. 4 is a perspective view of an electromagnetic field generation unit composed of two coil electromagnets.
FIG. 5 is a cross-sectional view of the electromagnetic field generation unit of FIG. 4.
FIG. 6 is a cross-sectional view of a permanent magnet (223) applied between a plurality of coil electromagnets.
FIG. 7 is a cross-sectional view of an electromagnetic field generation unit composed of a plurality of coil electromagnets.
FIG. 8 is an exemplary diagram of flow of a magnetic force when a plurality of electromagnetic field generation units are arranged in three dimensions.
FIG. 9 is a diagram of an AC signal applied to a coil electromagnet.
FIG. 10 is a logic diagram of an H-bridge circuit according to one embodiment of the present invention.
FIG. 11 is a computer-aided engineering (CAE) image of magnetic force transmission according to a repulsive force generated by an electromagnetic field generation unit.
FIG. 12 is a CAE image of magnetic force transmission according to an attractive force generated by an electromagnetic field generation unit.
FIG. 13 is a block diagram of a system for controlling a plurality of electromagnetic field generation units.
FIG. 14 is a perspective view of an electromagnet unit according to another embodiment of the present invention.
FIG. 15 is a cross-sectional view of the electromagnet unit according to another embodiment of the present invention.
FIG. 16 is a CAE image of magnetic force transmission according to a repulsive force generated by the electromagnet unit of FIGS. 14 and 15.
FIG. 17 is a CAE image of magnetic force transmission according to an attractive force generated by the electromagnet unit of FIGS. 14 and 15.
FIG. 18 is a cross-sectional view of a multilayer printed circuit board (PCB) pattern-type electromagnet unit according to another embodiment of the present invention.
FIG. 19 is a diagram illustrating an independent current-flow pattern of a multilayer PCB according to another embodiment of the present invention.
FIG. 20 is a diagram illustrating an integrated current-flow pattern (always inducing the same polarity) of the multilayer PCB according to another embodiment of the present invention.
FIG. 21 is a diagram illustrating an integrated current-flow pattern (always inducing a different polarity) of the multilayer PCB according to another embodiment of the present invention.
FIG. 22 is a diagram for describing a connection of an electrical conduction pattern for each layer of the multilayer PCB according to another embodiment of the present invention.
FIG. 23 is a conceptual diagram of an electromagnet unit that utilizes a permanent magnet and coils.
FIG. 24 is a cross-sectional view of the electromagnet unit that utilizes the permanent magnet and the coils.
FIG. 25 is a conceptual diagram of a pulsed electromagnetic field generation unit through permanent magnet rotation.
FIG. 26 is a conceptual diagram of a pulsed electromagnetic field limiting to which an enclosure is applied.
FIG. 27 is a voltage control waveform diagram according to another embodiment of the present invention.
FIG. 28 is a block diagram of a system for synchronizing electromagnet generation according to another embodiment of the present invention.
FIG. 29 is a flowchart illustrating a process for controlling a trigger switch unit.
FIG. 30 is a logic diagram of an H-bridge circuit according to another embodiment of the present invention.

### [Modes of the Invention]

Specific structural and functional descriptions of embodiments according to the concept of the present invention disclosed in this specification are only for the purpose of describing embodiments according to the concept of the present invention, and the embodiments according to the concept of the present invention may be embodied in various forms and are not to be construed as limited to the embodiments described in this specification.

While the embodiments according to the concept of the present invention may be modified in various ways and take on various alternative forms, specific embodiments thereof are shown in the drawings and described in detail in this specification. There is no intent to limit the embodiments according to the concept of the present invention to the particular forms disclosed. On the contrary, the present invention is to cover all modifications, equivalents, and alternatives falling within the spirit and scope of the appended claims.

It should be understood that, although the terms "first," "second," and the like may be used herein to describe various elements, the elements are not limited by the terms. The terms are only used to distinguish one element from another element. For example, a first element could be termed a second element, and, similarly, a second element could be termed a first element, without departing from the scope of the present invention.

It should be understood that when an element is referred to as being "connected" or "coupled" to another element, the element may be directly connected or coupled to another element or intervening elements may be present. In contrast, when an element is referred to as being "directly connected" or "directly coupled" to another element, there are no intervening elements present. Other words used to describe the relationship between elements should be interpreted in a like fashion (i.e., "between" versus "directly between," "adjacent" versus "directly adjacent," and the like.).

The terminology used herein is for the purpose of describing particular embodiments only and is not intended to be limiting to the present invention. As used herein, the singular forms "a" and "an" are intended to also include the plural forms, unless the context clearly indicates otherwise. It should be further understood that the terms "comprise," "comprising," "include," and/or "including" used herein specify the presence of stated features, integers, steps, operations, elements, parts, or combinations thereof, but do not preclude the presence or addition of one or more other features, integers, steps, operations, elements, parts, or combinations thereof.

Unless otherwise defined, all terms including technical and scientific terms used herein have the same meaning as commonly understood by one of ordinary skill in the art to which this invention belongs. It should be further understood that terms, such as those defined in commonly used dictionaries, should be interpreted as having a meaning that is consistent with their meaning in the context of the relevant art and are not to be interpreted in an idealized or overly formal sense unless expressly so defined herein.

In the drawings, the thicknesses of layers and regions are exaggerated for clarity. When a first layer is referred to as being "on" a second layer or substrate or is referred to as being bonded or adhered to the second layer or substrate, the firs layer may be formed directly on the second layer or substrate, or a third layer may be interposed therebetween. Portions indicated by the same reference numerals throughout the specification represent the same components.

Terms such as "upper end," "lower end," "upper surface," "lower surface," "front surface," "rear surface," "upper portion," "lower portion," and the like may be used to distinguish the relative positions of components from one another. For example, when an end, surface, or portion at an upper side in a drawing is named as an upper end, surface, or portion, and an end, surface, or portion at a lower side in the drawing is named as a lower end, surface, or portion for convenience, in reality, the upper end, surface, or portion may be named as the lower end, surface, or portion and the lower end, surface, or portion may be named as the upper end, surface, or portion without departing from the scope of the present invention. In addition, the components in the drawing are not necessarily drawn to scale, and for example, the sizes of some components in the drawing may be exaggerated compared to other components to aid understanding of the present invention.

Hereinafter, embodiments of the present invention will be described in detail with reference to the accompanying drawings.

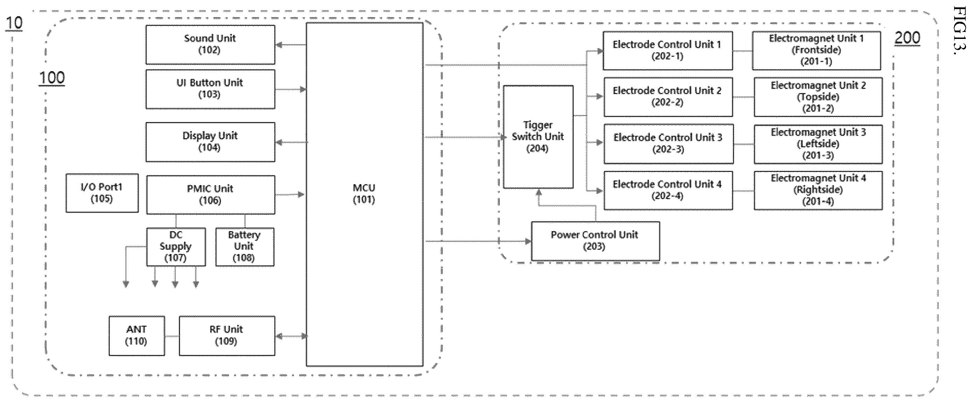

As illustrated in FIG. 13, a pulsed electromagnetic field generation unit 200 according to an embodiment of the present invention includes some or all of an electromagnet unit 201, an electrode control unit 202, a power control unit 203, and an electromagnet operation switch unit 204.

Here, the expression "include some or all" does not significantly differ from its previous meaning. The electromagnet unit 201 generates a magnetic field when a current is applied thereto, and the electrode control unit 202 changes a direction of the current applied to the electromagnet unit 201 to change a magnetic polarity of the electromagnet unit 201 to induce a waveform in the form of an AC. Further, the power control unit 203 controls the amount of voltage and current applied to the electromagnet unit 201 to control a magnitude of an electromagnetic force, and the electromagnet operation switch unit 204, that is, a trigger switch, functions as a switch that can start and stop a plurality of electromagnet units simultaneously. An On/Off time of the electromagnet operation switch unit 204 is controlled through a micro controller unit (MCU) 101 of a control unit 100 to implement the operating Hertz (Hz). The electromagnet unit 201 according to one embodiment of the present invention may include a plurality of electromagnet units and may include first to fourth electromagnet units 201-1 to 201-4. In consideration of a user's knees, the first to fourth electromagnet units 201-1 to 201-4 may each be provided to be positioned on upper, lower, left, and right sides. The pulsed electromagnetic field generation unit 200 operates to activate cells.

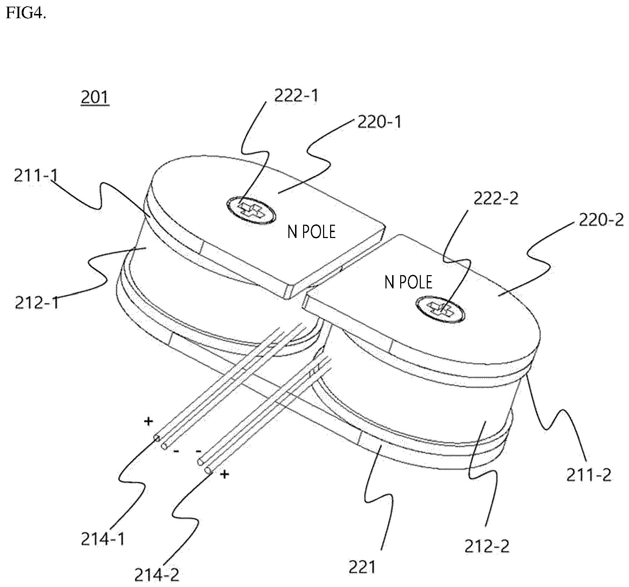

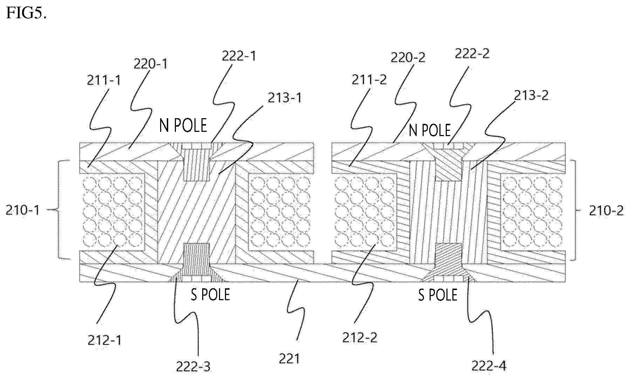

As illustrated in FIGS. 4 and 5, two or more coil electromagnets 210, that is, a plurality of electromagnets 210, may be provided, and thus the electromagnet unit 201 of the pulsed electromagnetic field generation unit 200 may transmit a magnetic force to a longer distance by applying repulsive and attractive forces of the coil electromagnets 210. Here, the longer distance may be defined as a distance exceeding a reference value. In this case, the electromagnet unit 201 may include a coil electromagnet 210 composed of a cylindrical frame 211 around which a winding coil 212 can be wound in a certain shape, a winding coil 212 made of a conductive material such as copper or the like to generate a magnetic field when a current is applied, a ferromagnetic core 213 positioned at a center of a coil to transmit the magnetic field generated from the winding coil in a certain direction, and a sheathed insulated cable 214 connected to both ends of the winding coil 212 to which currents of positive (+) and negative (-) polarity are applied, and may include an inner plate 220 of a ferromagnetic body for transmitting the magnetic force transmitted from the ferromagnetic core 213 toward a direction of a human joint to a long distance, an outer plate (or the other plate) 221 of a ferromagnetic body for increasing the magnetic force of the inner plate (or one plate) 220 by connecting cores 213 of the electromagnets in pair applied to the opposite surface of the inner plate 220, and further, a fixing body 222 such as a screw for fixing the inner plate 220 and the outer plate 221 to the core 213 of the electromagnet unit. Here, the inner side refers to an area of the user's body joint that comes into contact with (or is close to) the skin.

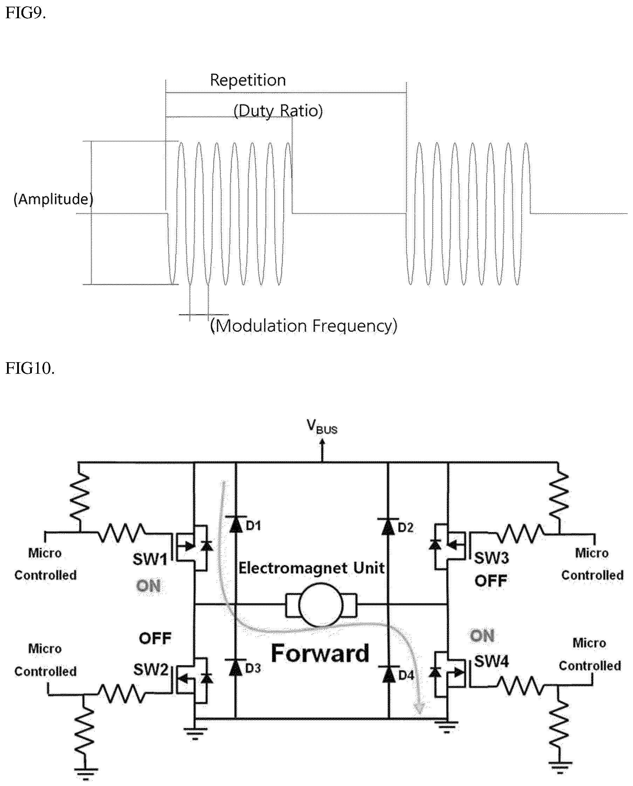

Inner plates 220 that connect the cores 213 of the coil electromagnets 210 in the electromagnet unit 201 are provided to be spaced by a close distance of 1 to 2 mm, for example, two plates are provided to be separated with a certain interval, and outer plates 221 that connect the cores 213 of the coil electromagnets 210 in the electromagnet unit 201 are connected as a single body at the shortest distance without a separation distance, and in order to transmit the magnetic force to a long distance, the form of the magnetic force has the characteristics of a pulse signal, and to this end, the outer plate 221 may include a unit 204 that generates a pulse signal and an electrode control unit 202 that changes a magnetic polarity in the form of an H-bridge. This is well illustrated in FIGS. 9 and 10. As illustrated in FIG. 10, the H-bridge is formed of first to fourth switching elements forming an H shape based on the electromagnet unit. Each switching element is formed of an NPN (or N-channel) element and a PNP (or P-channel) element of a transistor or a field-effect transistor (FET). In addition, the MCU 101 of FIG. 13 may alternately operate the switching elements by applying LOW and HIGH signals to the first switching element and the fourth switching element or by applying LOW and HIGH signals to the third switching element and the second switching element so that an opposite polarity is generated in the electromagnet unit. That is, it can be seen that the magnetic polarity is changed by operating the switching elements in a diagonal direction complementarily.

Further, the pulse applied to the electromagnet unit 201 to transmit the magnetic force to a long distance may be formed in the form of a carrier wave, and two or more electromagnet units 201 applied to the treatment device may be applied, a repulsive force may be generated on the inner plate 220 of one electromagnet unit and a repulsive force of the opposite polarity may be generated on the opposite electromagnet unit, and thus the magnetic force may be transmitted to a long distance. This is well illustrated in FIG. 9. Here, the expression "the pulse is in the form of a carrier wave" means, as illustrated in FIGS. 9 and 13, splitting a pulse signal (e.g., operating frequency) generated at a specified frequency and amplitude, more precisely, splitting a pulse in the pulse generation section to generate a new pulse to use the generated pulse. It can be seen that a preset pulse signal is modulated and used to transmit the magnetic force to a long distance and to penetrate light deeply. For example, in the embodiment of the present invention, a parasitic pulse signal of 20 to 50 Hz may be converted into a signal of about 2 MHz and used.

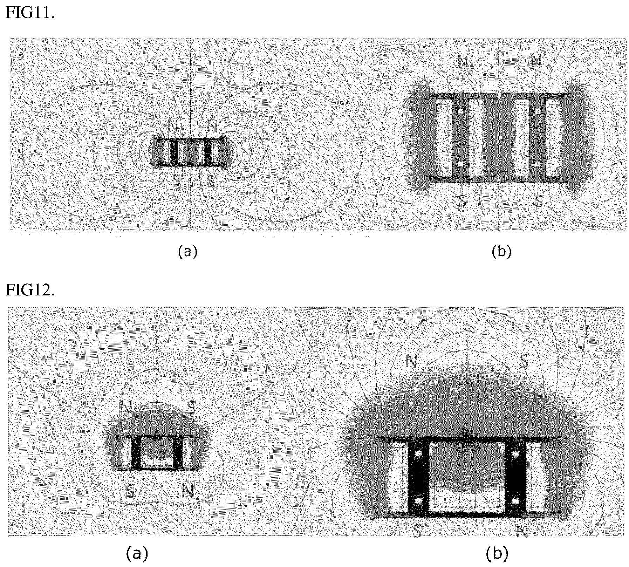

FIG. 9 is a diagram of an AC signal applied to an electromagnet unit, FIG. 10 is a logic diagram of an H-bridge circuit according to one embodiment of the present invention, FIG. 11 is a computer-aided engineering (CAE) image of magnetic force transmission according to a repulsive force generated by an electromagnet unit of FIG. 2, and FIG. 12 is a CAE image of magnetic force transmission according to an attractive force generated by the electromagnet unit.

The electrode control unit 202 is controlled to convert a pulse signal into an AC signal. The electrode control unit 202 is preferably formed as an H-bridge logic circuit as illustrated in FIG. 10, a direction of a current applied to the electromagnet unit 201 is changed under the control of the MCU 101, and thus a polarity of the inner plate 220 of the electromagnet unit 201 is also changed. Since the H-bridge circuit may operate in various forms according to the embodiment of the present invention, the present invention is not particularly limited to any one operating form (e.g., complementary operation).

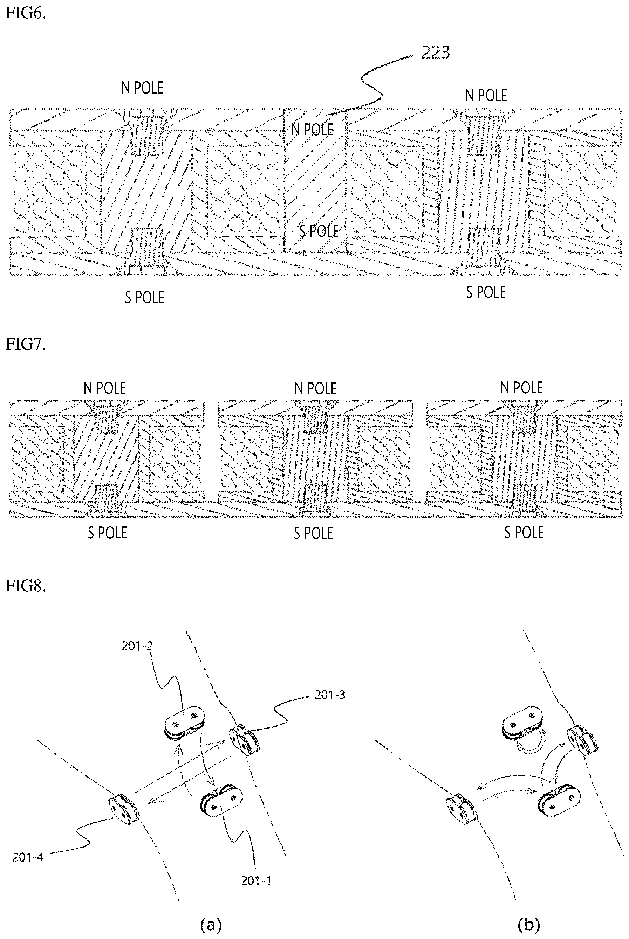

The electromagnet unit 201 is equipped with a plurality of coil electromagnets 210 therein. For example, as illustrated in FIGS. 6 and 7, two or more coil electromagnets 210-1 and 210-2 of multiple forms are applied to the electromagnet unit 201. The coil electromagnet 210 is wound several times in the form of a coil 212 around a cylindrical frame 211 of a non-magnetic material, and is connected to the electrode control unit 202 by applying a cable 214 with an insulating material covering such as polyvinyl chloride (PVC) to an end of the coil. It is preferable to use a non-magnetic material such as plastic as the material of the cylindrical frame 211.

Further, the ferromagnetic core 213 is applied at the center of the winding coil to perform a function of transmitting the magnetic field generated from the winding coil 212 in a certain direction. That is, according to the direction of the current applied to the insulating cable 214 connected to both ends of the winding coil 212, the magnetic polarities at both ends of the ferromagnetic core 213 are changed to N-S poles or S-N poles. The inner plate (or the one plate) 220 is connected to the both ends of the ferromagnetic core 213 of the paired coil electromagnets 210 with a slight distance in a direction of the human body using fixing bodies 222-1 and 222-2, and the outer plate (or the other plate) 221 is connected and fixed in an opposite direction of the inner plate using fixing bodies 222-3 and 222-4. Further, in order to transmit the magnetic force to a longer distance, a permanent magnet 223 may be applied between coil electromagnets as illustrated in FIG. 6, and a plurality of coil electromagnets may be connected and used as illustrated in FIG. 7.

The purpose is to transmit the magnetic force of the core 213 to the outer plates 221 and 222 to transmit the magnetic force to a long distance, and thus each plate and fixing body use a ferromagnetic material with a high relative magnetic permeability. Here, the relative magnetic permeability refers to a rate of magnetization, which is a rate compared to the magnetic permeability in a vacuum, that is, when this value is hundreds to thousands, it is called a ferromagnetic material.

As illustrated in FIG. 4, when a current with + and - polarities is actually applied to an insulated cable 214-1 of the coil electromagnet 210-1 positioned on the left side of the electromagnet unit 201 and a current with - and + polarities is applied to an insulated cable 214-2 of the right coil electromagnet 201-2, an N pole is induced in an inner plate 220-1 of the left coil electromagnet 210-1 and an N pole is induced in an inner plate 220-2 of the right coil electromagnet 210-2 according to Ampere's right-hand rule.

In this case, as illustrated in a CAE simulation analysis result of FIG. 11, directions of magnetic forces applied to two inner plates is the same, and thus a repulsive force occurs between the two inner plates, and this enables the magnetic force to be transmitted to a long distance.

When a current of + and - polarities is applied to the insulated cable 214-2 of the right coil electromagnet 210-2, an S pole is induced in the inner plate 220-2 of the right coil electromagnet, an attractive force is generated between the inner plates, and a mutually pulling force is generated. Even in the case in which such an attractive force is generated, as illustrated in a CAE simulation analysis result of FIG. 12, it can be seen that the magnetic force generated between the inner plates also reaches a long distance because each of cores 213-1 and 213-2 is connected through the outer plate 221.

The number and positions of the electromagnet units 201 may be applied differently and may be changed in various ways according to the anatomical characteristics of the treatment area. When the embodiment of the present invention is applied to the treatment of knee joint pain, it is preferable to position the electromagnet unit 201 as illustrated in FIG. 8 so that it can be directed toward the articular cartilage of a lower part of the femur and the meniscus of the tibia, where cartilage wear occurs and inflammation easily occurs.

By arranging the electromagnet unit 201 as described above and changing the magnetic force and polarity of each electromagnet unit 201, the magnetic force passes through the joint inflammation area in various directions and in various intensities as illustrated in FIG. 8.

In particular, it can be seen that when a current is applied so that a repulsive force is generated and an attractive force is generated between the electromagnet units 201 facing each other, the magnetic force reaches a longer distance.

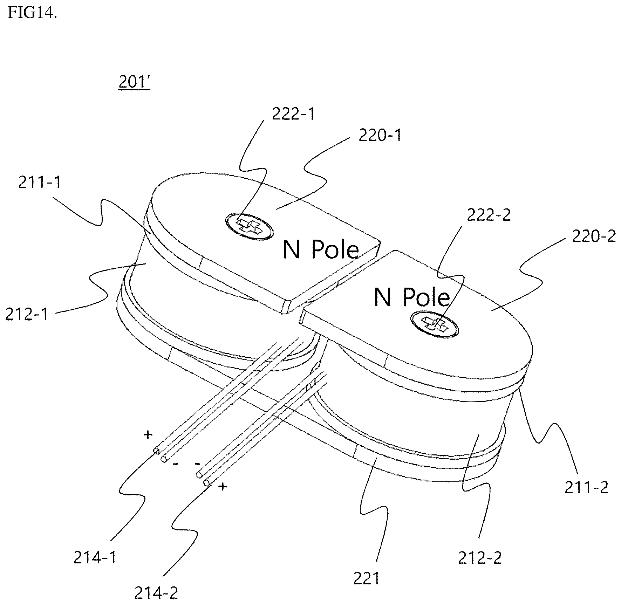

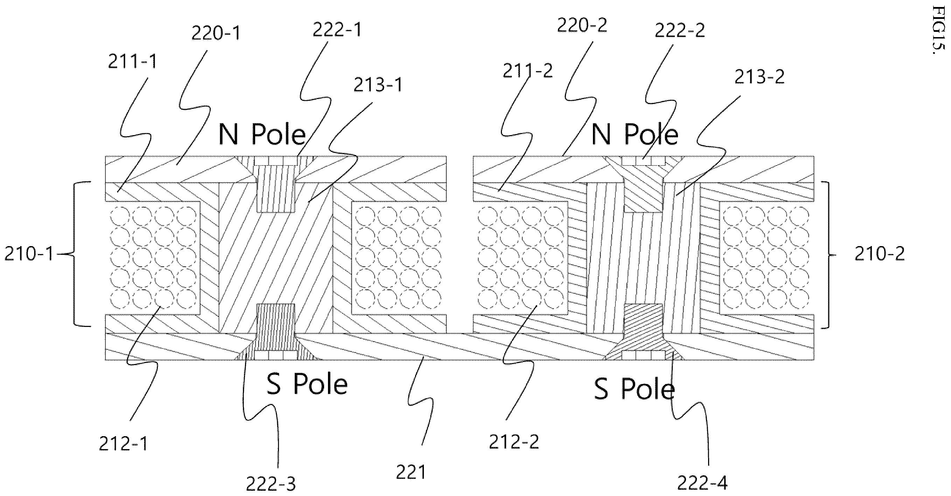

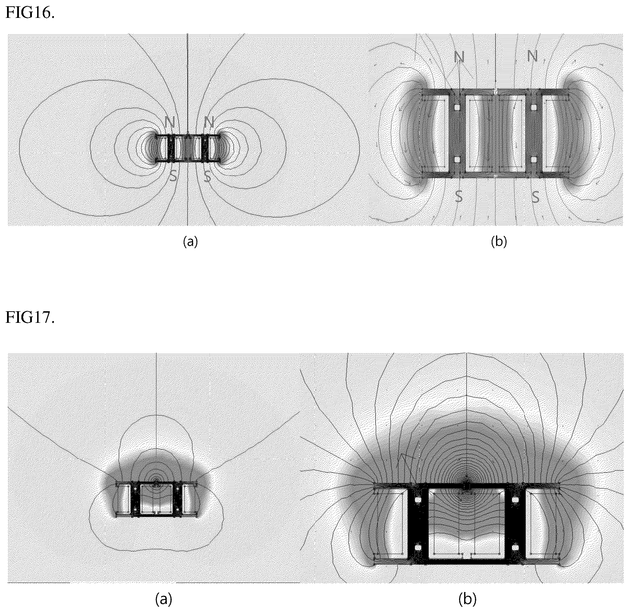

FIG. 14 is a perspective view of an electromagnet unit according to another embodiment of the present invention, FIG. 15 is a cross-sectional view of the electromagnet unit according to another embodiment of the present invention, FIG. 16 is a CAE image of magnetic force transmission according to a repulsive force generated by the electromagnet unit of FIGS. 14 and 15, and FIG. 17 is a CAE image of magnetic force transmission according to an attractive force generated by the electromagnet unit of FIGS. 14 and 15.

As illustrated in FIGS. 14 and 15, a configuration of an electromagnet unit 201' according to another embodiment of the present invention is not significantly different from that of the electromagnet unit 201 illustrated in FIGS. 4 and 5. However, in another embodiment of the present invention, it may be understood that the electromagnet unit 201 illustrated in FIGS. 4 and 5 is re-illustrated to explain the operation related to long-distance magnetic force transmission using a pair-type electromagnet. Therefore, the contents related to the electromagnet unit 201' according to another embodiment of the present invention are not significantly different from the electromagnet unit 201 illustrated in FIGS. 4 and 5, and thus the detailed contents will be replaced with those contents.

To briefly describe again, the electromagnet unit 201' may include a winding coil 212 that generates a magnetic force by applying a current thereto, a cylindrical frame 211 around which the winding coil can be wound in a certain shape, a winding copper coil 212 that generates a magnetic field when a current is applied, a ferromagnetic core 213 that is positioned at the center of the coil to transmit the magnetic field generated from the winding copper coil in a certain direction, a sheathed insulated cable 214 that is connected to both ends of the winding coil and to which the + of the DC power supply and the current are applied, a ferromagnetic inner plate 220 that is oriented in a direction of a human joint and transmits the magnetic force transmitted from the ferromagnetic core 213 to a long distance, a ferromagnetic outer plate 221 that is applied to an opposite surface of the ferromagnetic inner plate 220 and interconnects the cores 213 of the coil electromagnet 210 that are formed as a pair within the electromagnet unit 201 to increase a magnetic force of the inner plate, and a fixing body 222, such as a screw, for fixing the inner plate and the outer plate to the core 213 of the electromagnet unit.

In the case in which the magnetic field is generated by a pair-type electromagnet unit 201' of the same type as FIG. 14 and FIG. 15, when a magnetic force occurs between the electromagnets, the magnetic force may be transmitted to a longer distance, and when an attractive force occurs between the electromagnets, a range of the magnetic field may be controlled to a certain extent. That is, when the direction of the current applied to the coil is controlled to generate a repulsive force between the electromagnets, the same polarity is generated in the inner plate 220 and the outer plate 221 as illustrated in FIG. 16, and thus the magnetic force repulses each other, thereby enabling long-distance magnetic force transmission. Further, when the direction of the current applied to the coil is controlled to generate an attractive force between the electromagnets, opposite polarities are generated on the inner plate 220 and the outer plate 221, thereby limiting the range of magnetic force generation. The outer plate is not separated, which increases the magnetic force of the inner plate. It is preferable that the pair-type electromagnet unit 201' be provided as 2, 4, or 6 electromagnet units 201' in pairs to generate attractive and repulsive forces between each other. The inner plates 220 provided in pairs are spaced apart from each other at a certain distance, and the outer plates 221 connect the cores 213 in pair.

Through this, the problem that the magnetic force rapidly decreases with distance and a large amount of power is required to transmit the magnetic force to a long distance by configuring the coil in the form of a loop wire in the pulsed electromagnetic field treatment device applied to the pulsed electromagnetic field generation unit, which was mentioned as a conventional problem, may be solved (by winding multiple copper coils, applying current, and utilizing the electromagnetic force generated here). In addition, since the pulsed electromagnetic field generation unit applied to the pulsed electromagnetic field treatment device is in the form of a loop wire coil, the problem that the range of magnetic force generation cannot be limited may be solved. That is, in another embodiment of the present invention, in order to utilize the attractive and repulsive forces of the paired electromagnet units 201', the magnetic force may be transmitted to a long distance, or the magnetic force may be limited to a certain range as necessary.

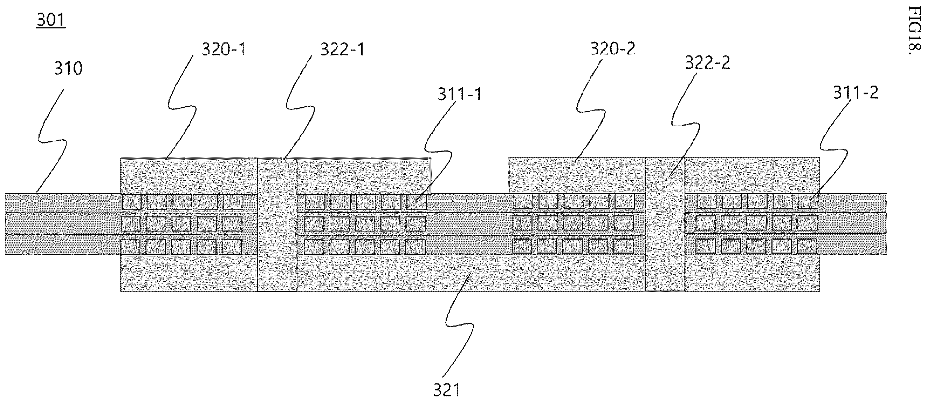

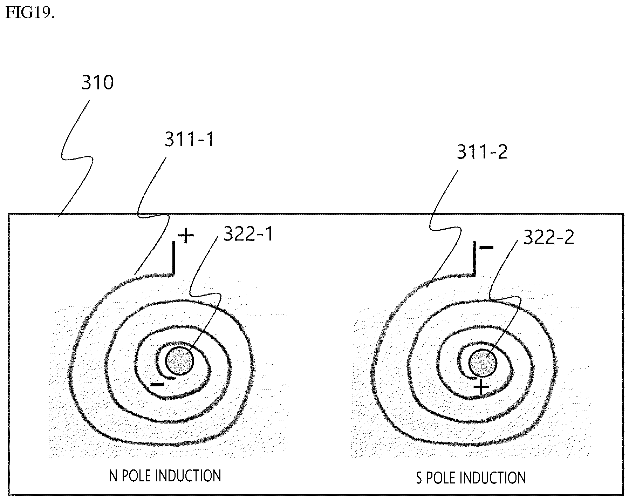

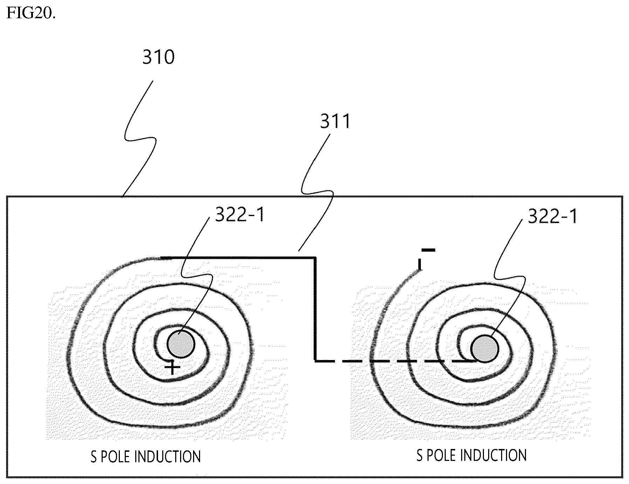

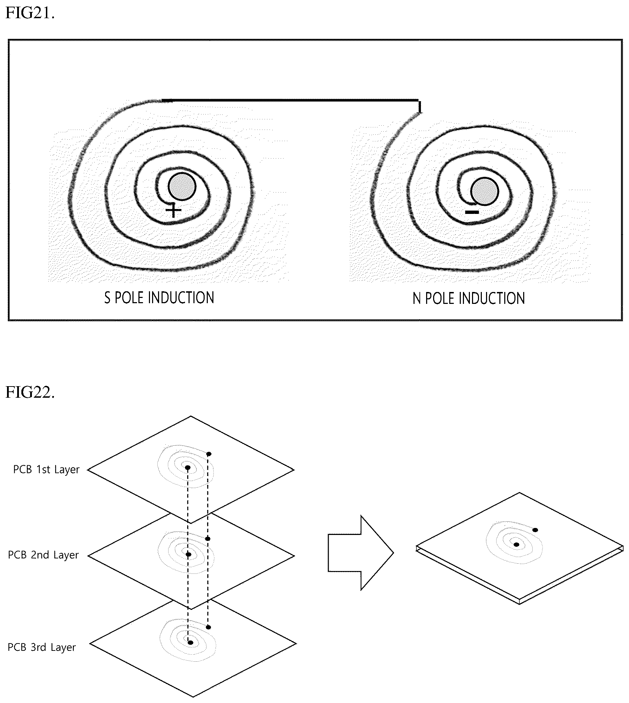

FIG. 18 is a cross-sectional view of a multilayer printed circuit board (PCB) pattern-type electromagnet unit according to another embodiment of the present invention, FIG. 19 is a diagram illustrating an independent current-flow pattern of a multilayer PCB according to another embodiment of the present invention, FIG. 20 is a diagram illustrating an integrated current-flow pattern (always inducing the same polarity) of a multilayer PCB according to another embodiment of the present invention, FIG. 21 is a diagram illustrating an integrated current-flow pattern (always inducing a different polarity) of a multilayer PCB according to another embodiment of the present invention, and FIG. 22 is a diagram for describing a connection of an electrical conduction pattern for each layer of a multilayer PCB according to another embodiment of the present invention.

In the case of pulsed electromagnetic medical devices with a small thickness and low magnetic strength, a magnetic force generation unit should be provided in a small size. To this end, in another embodiment of the present invention, current-flow patterns that conduct electricity in an inner layer of a multilayer PCB 310 are formed in a spiral curve manner to allow a current to flow, and thus a function of a small electromagnet unit may be implemented. When an inner plate 320 and an outer plate 321 are applied as in the pair-type electromagnet unit 201' described above, control through attractive and repulsive forces between the magnetic force generation units applied in pairs becomes possible.

The current-flow patterns provided in the multilayer PCB may be connected to each other to increase the strength of the magnetic force as illustrated in FIG. 22. In the general PCB manufacturing process, the patterns of layers are connected through via holes and plating processes. Generally, PCBs may increase the number of inner layers in the form of single-sided, double-sided, 4-layer, and 6-layer. The paired current-flow patterns may be independently formed as in FIG. 19 or connected to each other and integrated in a closed circuit manner as illustrated in FIGS. 20 and 21 according to the magnetic field polarity and purpose. When an independent current-flow pattern method is implemented as in FIG. 19, there is an advantage in that each polarity may be controlled as necessary, but there is a disadvantage in that the number of electromagnet units to be controlled increases. When an integrated current-flow pattern is applied as in FIGS. 20 to 21, there is a characteristic of always showing the same polarity or different polarity, but the number of electromagnet units to be controlled is reduced, and thus the load on the control unit may be reduced.

FIG. 18 illustrates a cross-section of a pair-type electromagnet unit using a multilayer PCB pattern according to another embodiment of the present invention. A PCB-type magnetic force generation unit 301 using a multilayer current-flow line may include a current-flow pattern line 311 implemented in a multilayer PCB (usually implemented with copper and having a spiral-shaped curved shape), a ferromagnetic inner plate 320 for directly transmitting a magnetic force transmitted from the current-flow pattern 311 toward a treatment area to a long distance, a ferromagnetic outer plate 321 applied to an opposite side of the ferromagnetic inner plate 320 and interlinking the magnetic force generated from the current-flow pattern 311 formed as a pair within the magnetic force generation unit 301 to increase a magnetic force of an inner plate, and a ferromagnetic core pin 322 positioned at the center of a coil for transmitting the magnetic field generated from the current-flow pattern 311 in a certain direction. In this case, the inner plate and the outer plate may be connected and fixed by the core pin.

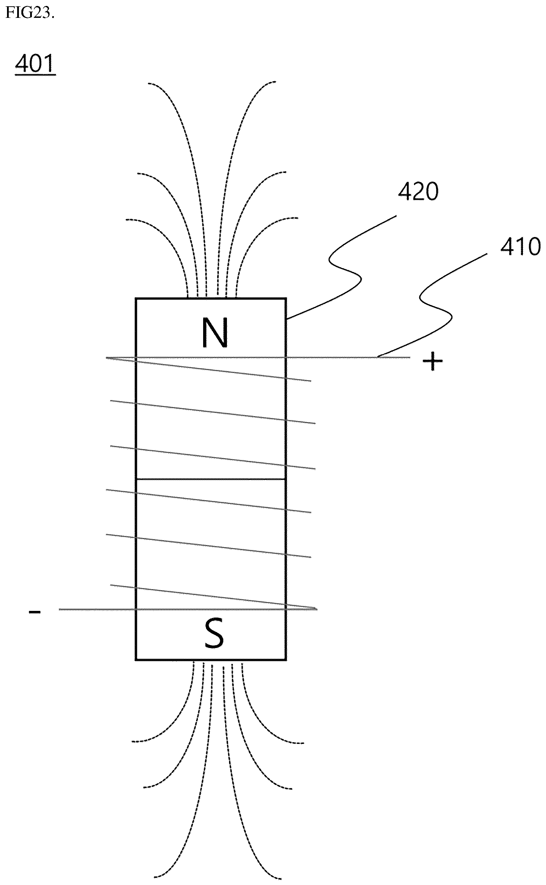

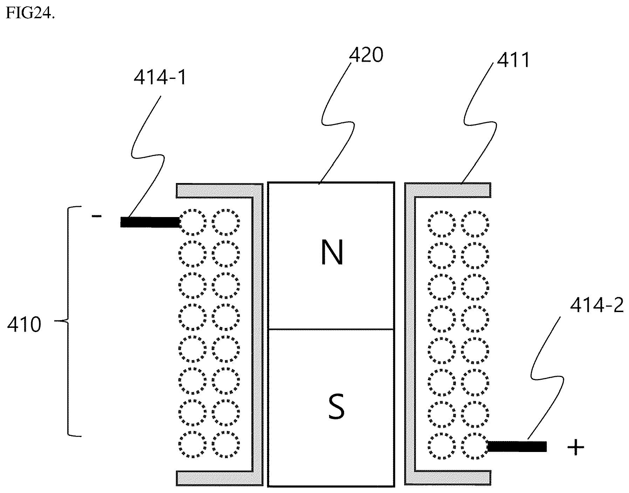

FIG. 23 is a conceptual diagram of an electromagnet unit that utilizes a permanent magnet and coils, and FIG. 24 is a cross-sectional view of the electromagnet unit that utilizes the permanent magnet and the coils.

In the case in which a pulsed electromagnetic field should be generated with low power using an electromagnet unit 401 utilizing a permanent magnet and coils, the permanent magnet-type electromagnet unit 401 to which a coil 410 for generating an electromagnetic field is applied may be applied to a permanent magnet 420 as illustrated in FIG. 23. Permanent magnets always generate a magnetic force of a certain strength, and an opposite polarity is generated by controlling the voltage as described above to generate an electromagnetic field. As a result, the strength of the magnetic force of the permanent magnet is changed to a certain frequency, and the treatment area is stimulated. A cylindrical frame 411 may be applied to the permanent magnet-type electromagnet unit 401 to wind the winding coil 410 in a certain shape, as illustrated in FIG. 24. It is preferably to apply a non-magnetic material such as plastic to the cylindrical frame 411. As necessary, a ferromagnetic metal material may be applied to the cylindrical frame 411. When a ferromagnetic metal material is applied, the strength and magnetic field area of the permanent magnet may be controlled more widely.

The permanent magnet-type electromagnet unit 401 according to another embodiment of the present invention may include a winding coil 410 that generates a magnetic force according to the current applied, a cylindrical frame 411 around which the winding coil can be wound in a certain shape, a sheathed insulated cable 414 that is connected to both ends of the winding coil and to which a + of a DC power source and a current are applied, and a permanent magnet 420 that constantly generates a magnetic force.

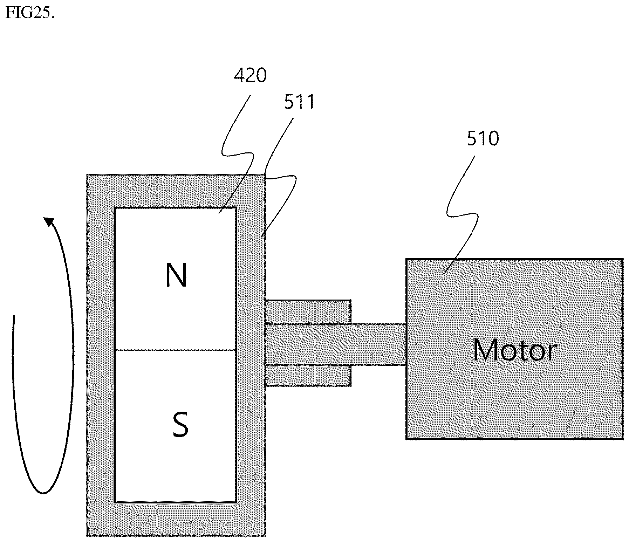

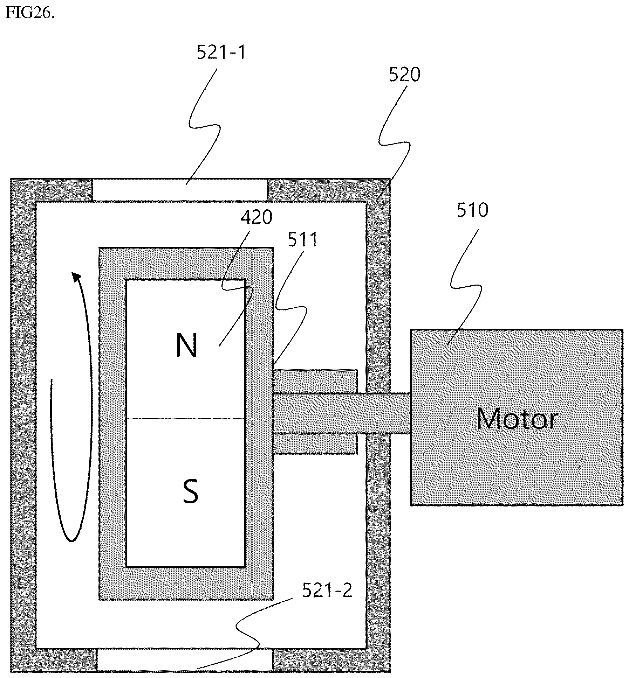

FIG. 25 is a conceptual diagram of a pulsed electromagnetic field generation unit through permanent magnet rotation, and FIG. 26 is a conceptual diagram of a pulsed electromagnetic field limiting to which an enclosure is applied.

A unit 501 for generating a pulsed electromagnetic field through permanent magnet rotation corresponds to a unit that rotates a permanent magnet at a constant RPM using a motor to generate a pulsed electromagnetic field with low power. It is desirable that the motor's RPM be tens to thousands of RPM so that a change in the pulsed electromagnetic field is several to several tens of Hz. A bracket made of a non-magnetic material such as plastic may be applied to connect the permanent magnet and the motor. Unlike the rotating permanent magnet, an enclosure 520 made of a ferromagnetic material that is fixed to the set and has an opening in a certain direction (treatment area) may be applied. This is well illustrated in FIG. 26. Through this, the transmission of a magnetic field to an unnecessary area may be blocked. The enclosure has one or more opening shapes 521, and it is preferable that an opening direction match a direction of the polarity of the permanent magnet.

More specifically, the unit 501 for generating the pulsed electromagnetic field through permanent magnet rotation may include a permanent magnet 420 that constantly generates a magnetic force, a motor 510 for rotating the permanent magnet to induce a change in magnetic force, a bracket 511 for fixing the permanent magnet and connecting the permanent magnet to a motor, an enclosure 520 made of a ferromagnetic material that has an opening area in a certain direction to control a diffusion direction of the pulsed electromagnetic field generated by the permanent magnet rotation, and an opening hole 521 through which the magnetic force can pass, as illustrated in FIGS. 25 and 26.

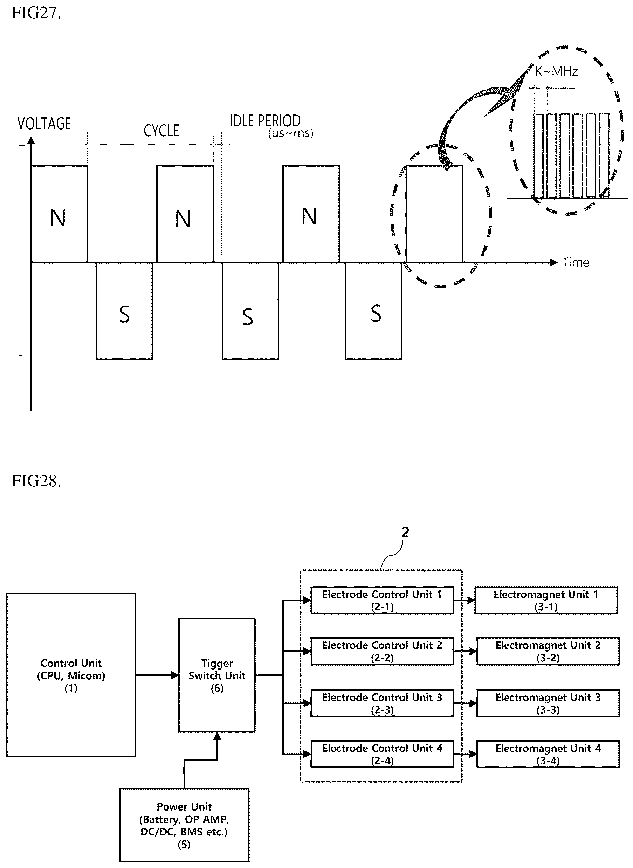

FIG. 27 is a voltage control waveform diagram according to another embodiment of the present invention.

As described above, the strength of an electric field € may be changed according to a voltage waveform applied to the electromagnetic field generation apparatus. When a waveform of a voltage/current applied to alternately change the polarity of the magnetic field at a constant Hz is continuously changed, energy efficiency decreases due to the generation of counter electromotive force at the inflection point. Therefore, in another embodiment of the present invention, the polarity of the voltage is changed at a constant cycle as illustrated in FIG. 27 to generate a pulsed electromagnetic field.

For example, when an N pole is expressed in a treatment area due to a current flow at the beginning (or at the minimum), the direction of the voltage is reversed after a certain period of time to alternately change the polarity of the treatment area to an S pole. In order to prevent counter electromotive force from occurring in the part where the polarity is changed, a pause of several µs to ms may be applied to a voltage inflection section as illustrated in FIG. 27. Further, while alternating the direction of the voltage at a certain period, a short-wave burst frequency (e.g., several KHz to MHz) may be applied to each section. In the embodiment of the present invention, when the direction of the voltage is changed at a certain period and a pulsed electromagnetic field is generated using a separate burst frequency, long-distance transmission with low power is possible.

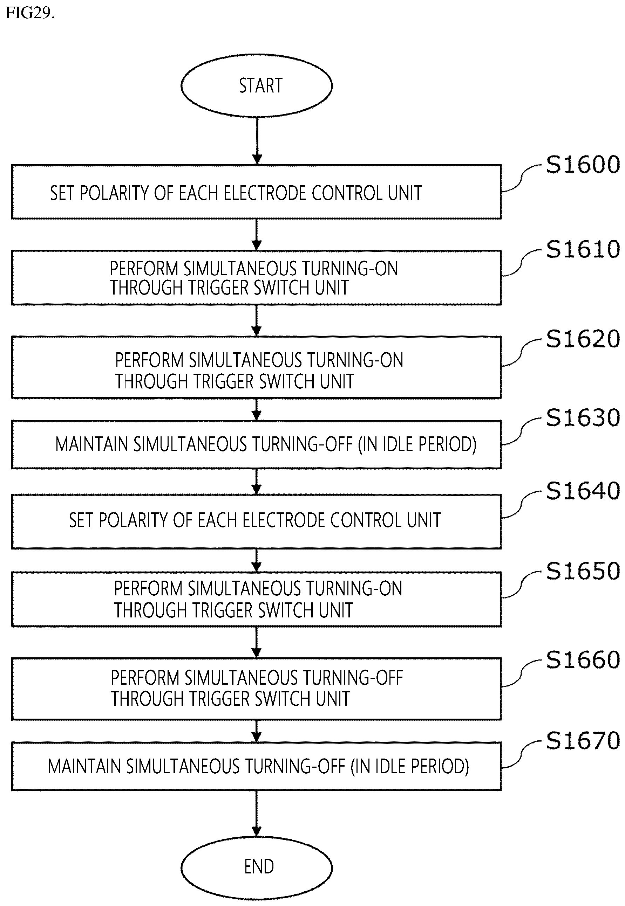

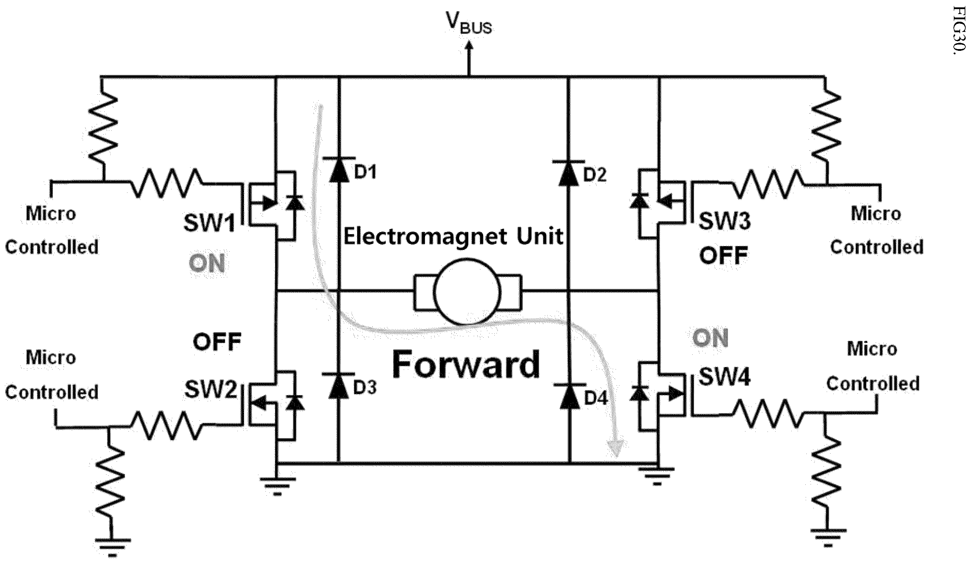

FIG. 28 is a block diagram of a system for synchronizing electromagnet generation according to another embodiment of the present invention, FIG. 29 is a flowchart illustrating a process for controlling a trigger switch unit, and FIG. 30 is a logic diagram of an H-bridge circuit according to another embodiment of the present invention.

As described above, when the synchronization of the pulsed electromagnetic field using a trigger switch is performed by controlling a plurality of electromagnet modules (here, the module may be an integrated circuit (IC) chip in which circuits are integrated, or a board in which the chip and its peripheral circuits are formed on a PCB, and the module may also be a component, element, device, etc.) with a single MICOM, an unsynchronized section occurs for each electromagnet, and thus the efficiency of superposition and cancellation between the magnetic forces generated from each electromagnet is reduced. In the embodiment of the present invention, as illustrated in FIG. 29, a trigger switch unit 6 may be added between a MICOM constituting a control unit 1 and electromagnet polarity control units 2 (2-1 to 2-4).

The trigger switch unit 6 serves to simultaneously set and operate the polarity of the polarity control units 2 (2-1 to 2-4). Through this, the plurality of polarity control units operate simultaneously and the output of the magnetic force of the electromagnet unit is also synchronized.

An operation of controlling the trigger switch unit 6 may be performed as illustrated in FIG. 29. In particular, the trigger switch unit may be controlled in consideration of an idle period of the input voltage described above. More specifically, an operation in which a frequency of a pulsed electromagnetic field is controlled using the trigger switch unit 6 or the trigger switch unit may include an operation S1600 of setting the polarity of each electrode control unit, an operation S1610 of performing simultaneous turning-on through the trigger switch unit, an operation S1620 of performing simultaneous turning-off through the trigger switch unit, an operation S1630 of maintaining simultaneous turning-off (in an idle period), an operation S1640 of setting the polarity of each electrode control unit, an operation S1650 of performing simultaneous turning-on through the trigger switch unit, an operation S1660 of performing simultaneous turning-off through the trigger switch unit, and an operation S1670 of maintaining simultaneous turning-off (in an idle period).

FIG. 30 illustrates the H-bridge logic circuit diagram, and it may be understood that a re-drawing of FIG. 10 for convenience of description. Therefore, the detailed information related to FIG. 30 will be replaced with the contents described in FIG. 10. Briefly, the H-bridge is formed with first to fourth switching elements forming an H shape based on the electromagnet unit. Each switching element is formed with an NPN (or N-channel) element and a PNP (or P-channel) element of a transistor or FET. In addition, the MCU 101 of FIG. 13 may alternately operate the switching elements by applying LOW and HIGH signals to the first switching element and the fourth switching element or by applying LOW and HIGH signals to the third switching element and the second switching element so that an opposite polarity is generated in the electromagnet unit. That is, it can be seen that the magnetic polarity is changed by operating the switching elements in a diagonal direction complementarily.

In addition to the above, the pulsed electromagnetic field generation module (or device or the like) applied to the pulsed electromagnetic field treatment device according to another embodiment of the present invention may perform various operations, and since other detailed information has been sufficiently described above, it will be replaced with that description.

While exemplary embodiments of the present invention have been illustrated and described, the present invention is not to be construed as limited to the particular embodiments described above, and it will be understood that various modifications may be made without departing from the spirit and scope of the present invention and such modifications are not individually understandable from the present invention.

On the other hand, even if it is described that all constituent elements that constitute an exemplary embodiment of the present invention are coupled into one or coupled to operate, the present invention is not essentially limited to such an exemplary embodiment. That is, within the purpose range of the present invention, all the constituent elements may be selectively coupled into one or more to perform operation. Further, although each of the constituent elements may be implemented by independent hardware (e.g., a hardware processor), a part or the whole of the constituent elements may be selectively combined and implemented as a computer program having a program module that performs functions of a part or the whole of one or a plurality of combined hardware configurations. Codes and code segments that constitute the computer program may be easily reasoned by those skilled in the art to which the present invention pertains. Such a computer program may be stored in a non-transitory computer readable medium to be read and executed by the computer to implement an exemplary embodiment of the present invention.

Here, the non-transitory computer readable medium is not a medium that stores data for a short period, such as a register, a cache, or a memory, but means a medium which semi-permanently stores data and is readable by a device. Specifically, various applications and programs as described above may be stored and provided in the non-transitory computer readable medium, such as, a compact disc read-only memory (CD-ROM), a digital video disc (DVD), a hard disc, a Blu-ray disc, a Universal Serial Bus (USB), a memory card, and a read-only memory (ROM).

The foregoing exemplary embodiments and advantages are merely exemplary and are not to be construed as limiting the present invention. The present teaching can be readily applied to other types of apparatuses. Also, the description of the exemplary embodiments of the present invention is intended to be illustrative, and not to limit the scope of the claims, and many alternatives, modifications, and variations will be apparent to those skilled in the art.

## Claims

1. A pulsed electromagnetic field generation apparatus comprising:
an electromagnet unit configured to generate attractive and repulsive forces between a plurality of coil electromagnets, each of which is formed by winding a coil around a core, and adjust a magnetic force (F) generated by the plurality of coil electromagnets; and
an electromagnet operation switch unit configured to adjust at least one of a voltage and frequency applied to each of the plurality of coil electromagnets.

2. The pulsed electromagnetic field generation apparatus of claim 1, wherein the electromagnet unit includes:
a first electromagnet unit which includes a pair of a first coil electromagnet and a second coil electromagnet and generates attractive and repulsive forces between the first coil electromagnet and the second coil electromagnet; and
a second electromagnet unit which includes a pair of a third coil electromagnet and a fourth coil electromagnet, generates attractive and repulsive forces between the third coil electromagnet and the fourth coil electromagnet, and generates attractive and repulsive forces between the first electromagnet unit and the second electromagnet unit.

3. The pulsed electromagnetic field generation apparatus of claim 2, further comprising an electrode control unit configured to change a magnetic polarity by changing a direction of a current applied to each of the first electromagnet unit and the second electromagnet unit, and control the magnetic polarity to be changed in an AC form.

4. The pulsed electromagnetic field generation apparatus of claim 1, wherein the electromagnet operation switch unit includes a trigger switch that simultaneously turns on or off the first electromagnet unit and the second electromagnet unit.

5. The pulsed electromagnetic field generation apparatus of claim 1, wherein the electromagnet unit includes:
one plate formed of a plurality of plates separated at one side of the first coil electromagnet and the second coil electromagnet; and
the other plate formed of a single plate formed by connecting one side surfaces of the first coil electromagnet and the second coil electromagnet to each other at the other side of the first coil electromagnet and the second coil electromagnet.

6. The pulsed electromagnetic field generation apparatus of claim 5, wherein the electromagnet unit includes a permanent magnet that is provided between the one plate of the first coil electromagnet and the one plate of the second coil electromagnet to transmit the magnetic force to a long distance.

7. The pulsed electromagnetic field generation apparatus of claim 1, wherein the plurality of coil electromagnets are formed using a conductive pattern formed on a multilayer printed circuit board (PCB).

8. A method of driving a pulsed electromagnetic field generation apparatus, comprising:
generating, by an electromagnet unit, attractive and repulsive forces between a plurality of coil electromagnets, each of which is formed by winding a coil around a core, and adjusting a magnetic force (F) generated by the plurality of coil electromagnets; and
adjusting, by an electromagnet operation switch unit, at least one of a voltage and frequency applied to each of the plurality of coil electromagnets.

9. The method of claim 8, wherein the adjusting of the magnetic force includes:
generating, by a first electromagnet unit including a pair of a first coil electromagnet and a second coil electromagnet, attractive and repulsive forces between the first coil electromagnet and the second coil electromagnet; and
generating, by a second electromagnet unit including a pair of a third coil electromagnet and a fourth coil electromagnet, attractive and repulsive forces between the third coil electromagnet and the fourth coil electromagnet, and generating attractive and repulsive forces between the first electromagnet unit and the second electromagnet unit.

10. The method of claim 9, further comprising changing, by an electrode control unit, a magnetic polarity by changing a direction of a current applied to each of the first electromagnet unit and the second electromagnet unit, and controlling the magnetic polarity to be changed in an AC form.

11. The method of claim 8, wherein the adjusting of the at least one of the voltage and frequency includes simultaneously turning on or off, by a trigger switch constituting the electromagnet operation switch unit, the first electromagnet unit and the second electromagnet unit.

12. The method of claim 8, wherein the electromagnet unit includes:
one plate formed of a plurality of plates separated at one side of the first coil electromagnet and the second coil electromagnet; and
the other plate formed of a single plate formed by connecting one side surfaces of the first coil electromagnet and the second coil electromagnet to each other at the other side of the first coil electromagnet and the second coil electromagnet.

13. The method of claim 12, wherein the electromagnet unit includes a permanent magnet that is provided between the one plate of the first coil electromagnet and the one plate of the second coil electromagnet to transmit the magnetic force to a long distance.

14. The method of claim 8, wherein the plurality of coil electromagnets are formed using a conductive pattern formed on a multilayer printed circuit board (PCB).
